# EUROPEAN PATENT APPLICATION

(11) **EP 4 609 829 A1**
(43) Date of publication of application: **03.09.2025**
(21) Application number: 23881925.4
(22) Date of filing: 26.10.2023
(51) Int. Cl.: A61F 2/24

(54) **CARDIAC IMPLANT AND CARDIAC IMPLANT SYSTEM**

(30) Priority: 27.10.2022 CN 202211330036
(71) Applicant: United Innomed (Shanghai) Limited, Shanghai 201315 (CN)
(72) Inventor: WANG, Li, Shanghai 201315 (CN)
(74) Representative: Winter, Brandl - Partnerschaft mbB
(86) International application number: PCT/CN2023/126897
(87) International publication number: WO 2024/088350

(57) **Abstract**

A cardiac implant and an implantation system therefor. The cardiac implant includes a treatment device and at least one sensor. The treatment device is used for reconstructing the function of the valve to prevent blood regurgitation from a second chamber to a first chamber. The at least one sensor is attached to the treatment device, and used for sensing physiological information of a patient. The cardiac implant not only mitigates the regurgitation condition of the patient so as to achieve a therapeutic purpose, but also senses the physiological information of the heart of the patient so as to achieve a monitoring purpose. By using the cardiac implant, the patient does not need to or seldom needs to go to a hospital for regular reexamination, so that the financial burden and time cost of the patient can be reduced.

## Description

### TECHNICAL FIELD

The present application relates to the field of medical apparatus and instruments technologies, and in particular, to a cardiac implant and an implant system.

### BACKGROUND

A heart valve refers to a valve located between an atrium and a ventricle or a valve located between the ventricle and an artery. The valve plays a critical role in the heart' ceaseless blood circulation. After blood flows through, the valve closes to prevent blood regurgitation. Various structural factors may affect proper closure of the heart valve, and thereby inducing the blood regurgitation. Taking a mitral valve as an example, if the mitral valve fails to close properly, the blood may flow from the left ventricle to the left atrium through the mitral valve during systole, and thereby impairing a patient's health.

To improve the issue of blood regurgitation, some implants suitable for implanting in a patient's heart have been developed. These implants may be delivered between leaflets of a patient's valve through operation to improve the condition of valve closure, and thereby reducing the likelihood of blood regurgitation. However, a current implant, including its support structure, often affects the structure of an existing valve while repairing and improving closure of the valve, and thereby resulting in some hemodynamics and/or clinical issues.

After the implant being implanted into the patient's heart, the patient usually needs to go to the hospital regularly for follow-up examination. In general, there are two aspects of purposes of a regular follow-up examination. On one aspect, it is to observe the effect of the implant on the cardiac function (whether it has improved, and the like) and the operating condition of the implant to determine whether there are any adverse events associated with the implant. On the other hand, it is to check the overall condition of the patient, such as heart failure and the use of the medicine, and the like, so that it is determined whether the condition of the patient is effectively improved or whether further deterioration occurs. These follow-up examinations are generally required to be performed in the hospital (or clinic). Frequent follow-up examinations, especially for a patient in remote areas, will cause the economic burden and time cost of the patient to be increased. At the same time, since the follow-up time has a relatively long interval, the change of the condition is often unpredictable, and thereby making it more difficult to achieve the goals of identifying problems early, preventing the condition from worsening and improving the quality of life for patients.

### SUMMARY

In view of the above, in a first aspect, the present application provides a cardiac implant and an implant system.

In a first aspect, the present application provides a cardiac implant adapted to be implanted in a patient's heart. The heart includes a first chamber, a second chamber and a valve. The valve is configured to periodically allow blood to flow from the first chamber to the second chamber. The cardiac implant includes a treatment device and at least one sensor. The treatment device is used for repairing the function of the valve to prevent the blood from flowing back from the second chamber to the first chamber, and the at least one sensor is attached to the treatment device and configured to sense a physiological information of the patient.

In a possible implementation, the valve includes at least two leaflets, the treatment device includes a pad, the pad is configured to be located between the at least two leaflets and cooperate with the at least two leaflets to periodically open and close the valve, and the at least one sensor is attached to the pad.

In a possible implementation, the pad has a top surface configured to face the first chamber, the at least one sensor includes a pressure sensor, and the pressure sensor is attached to the top surface of the pad to sense a blood pressure in the first chamber; and/or, the pad has a bottom surface configured to face the second chamber, the at least one sensor includes a pressure sensor, and the pressure sensor is attached to the bottom surface of the pad to sense a blood pressure in the second chamber.

In a possible implementation, the treatment device also includes a support member or a fixing member connected to the pad, the fixation member is configured to attach the pad to one of the at least two leaflets, and the support member is configured to position the pad between the at least two leaflets.

In a possible implementation, the valve includes at least two leaflets, the treatment device includes a support member and a pad, the support member is configured to be located in the first chamber and be connected to the pad to position the pad between the at least two leaflets, the at least one sensor includes a pressure sensor, and the pressure sensor is attached to the support member.

In a possible implementation, the pad is movably connected to the support member; or, the pad is biased at a side of the support member to maintain fitting with one of the at least two leaflets of the valve; or, the pad is centrally positioned at a side of the support member to be centrally located between the at least two leaflets.

In a possible implementation, the pad and the pressure sensor are both located on a side of the support member closer to the second chamber in a direction from the first chamber to the second chamber, and the pressure sensor is provided on the support member and is offset from the pad in a radial direction of the support member.

In a possible implementation, an end of the pressure sensor is fixed to the support member, and the other end of the pressure sensor has a sensing surface, the sensing surface is configured to face the second chamber to sense a pressure of the blood flowing from the second chamber into the first chamber.

In a possible implementation, the cardiac valve includes at least two leaflets, the treatment device includes a coaptation member and at least two blades located on two sides of the coaptation member respectively, and the at least two blades are configured to position the coaptation member between the at least two leaflets.

In a possible implementation, the coaptation member has a top surface configured to face the first chamber, the at least one sensor includes a pressure sensor, the pressure sensor is attached to the top surface of the coaptation member to sense a blood pressure in the first chamber; and/or, the treatment device has a bottom surface configured to face the second chamber, the at least one sensor includes a pressure sensor, and the pressure sensor is attached to the bottom surface of the treatment device to sense a blood pressure in the second chamber.

In a possible implementation, the at least one sensor includes a pressure sensor, and a sensing surface of the pressure sensor is configured to face the first chamber or the second chamber.

In another aspect, the present application also provides an implant system. The implant system includes the cardiac implant above-mentioned and an external device. The at least one sensor includes a wireless transmission unit, the external device includes a wireless reception unit, and the wireless reception unit receive pressure value information from the wireless transmission unit of the at least one sensor.

In a possible implementation, the external device also includes an analysis unit and an alert unit; the analysis unit receives the pressure value information from the wireless reception unit and determines whether to send an alert signal to the alert unit based on the pressure value information; and after the alert unit receives the alert signal, the alert unit sends an alert message.

The cardiac implant provided by the present application is not only able to improve the patient's regurgitation condition to achieve the purpose of therapeutic, but also is able to sense the physiological information of the patient's heart to achieve the purpose of monitoring. Because the sensor is able to provide hemodynamic information (such as a pressure) in the patient's atria and/or ventricle (especially a left atrium and/or a left ventricle), so that it is possible for a doctor to remotely access this information as needed. By using the cardiac implant provided by the present application, the patient may not need to or may need to visit the hospital for regular follow-ups less frequently, and the patient's condition is able to be known early, including the effectiveness of the implant and/or changes in the course of disease, so that early detection, diagnosis, and treatment is achieved. It can be seen that, the cardiac implant provided by the present application is able to reduce the economic burden and time cost for the patient. Moreover, in the present application, the sensor is positioned in the patient's heart by being attached to the treatment device, rather than being directly anchored in the patient's cardiac tissue, so that damage of a tissue is reduced.

### BRIEF DESCRIPTION OF THE DRAWINGS

In order to more clearly illustrate technical solutions of embodiments of the present application, the drawings to be used in the embodiments will be introduced briefly below.

It should be understood that, the drawings below merely show some embodiments of the present application, but not all the embodiments.

It should be understood that, the same or similar reference numerals are used in the drawings for representing the same or similar elements.

It should be understood that, the drawings are merely illustrative, and dimensions and proportions of the elements in the drawings are not necessarily accurate.
FIG. 1 is a schematic structural diagram of a cardiac implant according to an embodiment of the present application.
FIG. 2 is a schematic structural diagram illustrating the cardiac implant of FIG. 1 located in a patient's heart.
FIG. 3 is a schematic structural diagram illustrating a cardiac implant according to another embodiment of the present application that is located in a patient's heart.
FIG. 4 is a schematic structural diagram illustrating a cardiac implant according to yet another embodiment of the present application that is located in a patient's heart.
FIG. 5 is a schematic structural diagram illustrating a cardiac implant according to still another embodiment of the present application that is located in a patient's heart.
FIG. 6 is a schematic structural diagram illustrating a cardiac implant according to further another embodiment of the present application that is located in a patient's heart.
FIG. 7 is a schematic structural diagram of an implant system according to an embodiment of the present application.

### DETAILED DESCRIPTION OF THE EMBODIMENTS

Technical solutions of implementations provided by the present application will be described exemplarily with reference to the drawings of the implementations provided by the present application below. Apparently, the described implementations are merely a portion of the implementations of the present application, rather than all of them.

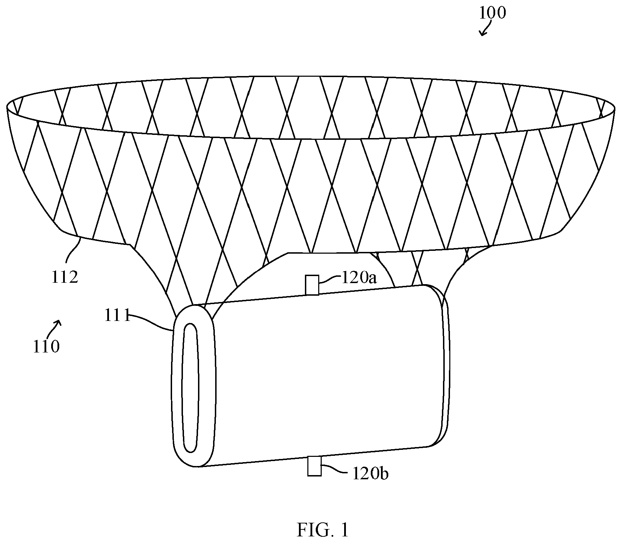

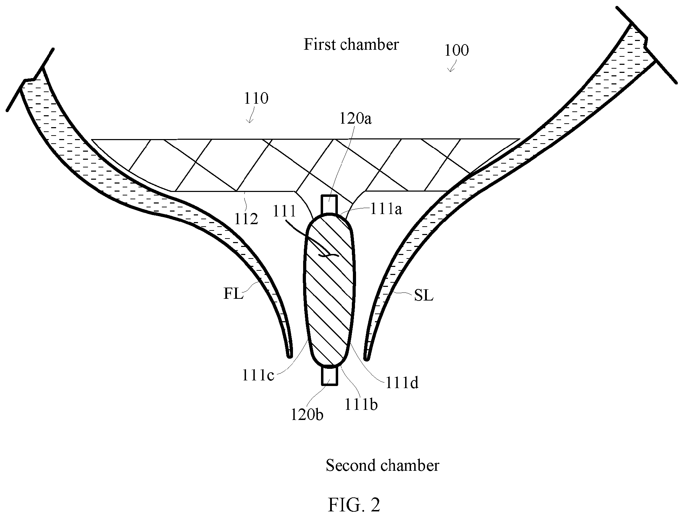

A cardiac implant 100 according to an embodiment of the present application will be described with reference to FIG. 1 and FIG. 2 below. FIG. 1 is a schematic structural diagram of the cardiac implant 100, and FIG. 2 illustrates a schematic structural diagram of the cardiac implant 100 when it is implanted at a patient's heart valve.

It should be noted that, in the drawings of the present application, the valve is located between a first chamber and a second chamber. The valve periodically allows blood to flow from the first chamber to the second chamber while preventing flowing in the reverse direction. The valve includes a pair of leaflets FL, SL matching with each other.

Specifically, the valve may be a mitral valve. Correspondingly, the first chamber may be the left atrium, the second chamber may be the left ventricle, and one of the pair of leaflets FL, SL is the anterior leaflet and the other is the posterior leaflet. It may be understood that, the cardiac implant provided by the present application is not limited to the application in mitral valve, but may also be applied to other heart valve (for example, a tricuspid valve), in this case, the heart valve includes three leaflets.

Referring to FIG. 1 and FIG. 2, the cardiac implant 100 includes a treatment device 110 and a pair of sensors 120a, 120b. It may be understood that, in other examples, the cardiac implant 100 may include only one sensor or three or more sensors. The treatment device 110 is configured to repair a function of the valve to prevent blood from flowing from the second chamber back to the first chamber. The implementation of the treatment device 110 will be described in detail below. The pair of sensors 120a, 120b are attached to the treatment device 110 and are configured to sense physiological information of the patient.

In an example, the physiological information may include, but is not limited to, one or more type of the following: blood pressure information, blood flow velocity information, blood pH information, blood temperature information, blood oxygen level information, electrocardiographic information, heart sound information, cardiac acceleration information and cardiac contractility information of the patient's heart. Correspondingly, the sensors in the present application may include any sensing device that is capable of sensing one or more type of the above-mentioned information.

The cardiac implant provided by the present application is not only able to improve the regurgitation of the patient for realizing a therapeutic purpose, but also is able to sense the physiological information of the heart of the patient for realizing a monitoring purpose. By using the cardiac implant provided by the present application, the patients may need to visit the hospital for follow-ups less frequently or not at all. Consequently, the cardiac implant provided by the present application is able to effectively reduce the financial burden and time cost for the patient. Furthermore, in the present application, at least one sensor is positioned in the patient's heart by attaching to the treatment device rather than being directly anchored to a tissue of the heart of the patient, and thereby harm to the tissue is reduced.

In a healthy heart, the pair of leaflets FL, SL are able to naturally coaptate and separate with the cardiac cycle to periodically close and open the valves, and thereby allowing the blood to flow from the first chamber to the second chamber while preventing flow in the opposite direction. However, for patients with valve insufficiency, when the valve is required to close, the pair of leaflets FL, SL fail to achieve proper coaptation, so that there is a gap between them, thereby the blood may flow through the valve from the second chamber back to the first chamber, and thus resulting in the regurgitation.

Referring again to FIG. 1 and FIG. 2, the treatment device 110 includes a pad 111. When the cardiac implant 100 is implanted in the patient's heart, the pad 111 is configured to be located between the pair of leaflets FL, SL. The pad 111 is configured to cooperate with the pair of leaflets FL, SL to enable the valve to be periodically opened and closed, and thereby allowing the blood to flow from the first chamber to the second chamber while preventing flowing in the reverse direction. Certainly, in an applications scenario of the tricuspid valve, the pad 111 may be configured to be located among three leaflets. A pair of collection devices (for example, sensors) 120a, 120b are attached to the pad 110 to facilitate fixing of the pair of collection devices 120a, 120b.

Referring again to FIG. 1 and FIG. 2, the pad 111 includes a top surface 111a facing the first chamber and a bottom surface 111b facing the second chamber. The pair of sensors 120a and 120b may be a pair of pressure sensors 120a, 120b. The pair of pressure sensors 120a, 120b may be respectively attached to the top surface 111a and bottom surface 111b of the pad 111 to sense blood pressure information of the first chamber and the second chamber, respectively.

Sensing surfaces (for example, vibrating diaphragms) of the pair of pressure sensors 120a, 120b face the first chamber and the second chamber, respectively, and are able to sense blood pressures in the first chamber and the second chamber of the entire cardiac cycle, respectively. This provides the doctor with hemodynamic information having diagnostic value. This information is critical for evaluating the overall cardiac function and the working condition of the valve and the implant. Meanwhile, the pressure information is also able to assist the doctor in evaluating the placement position of the implant during the implantation process and making real-time assessment of whether there is any obstruction to the blood flow into the second chamber during diastole. Therefore, the success of the implantation operation is able to be better ensured, and a better clinical effect is achieved.

Additionally, when the valve opens, the blood flow that flows from the first chamber to the second chamber is able to nearly directly impact onto the pressure sensor 120a (on its vibrating diaphragm) attached to the top surface 111a. This allows the physiological information (that is, blood pressure information) sensed by the pressure sensor 120a to reflect whether the blood flow that flows from the first chamber to the second chamber is normal, so that it is able to relatively accurately reflect whether there is a stenosis issue with the valve of the patient after the cardiac implant 100 is implanted.

When the valve closes, if there is still the regurgitation that flows from the second chamber to the first chamber, the regurgitation will nearly directly impact onto the vibrating diaphragm of the pressure sensor 120b attached to the bottom surface 111b, so that the physiological information (that is, pressure information) and variations thereof sensed by the pressure sensor 120b due to the blood flow will reflect whether there is the regurgitation that flows from the second chamber to the first chamber.

It may be understood that, when the pressure sensor is configured to side face sideways to the first chamber and second chamber, the blood flow will impact the sensing surface of the pressure sensor from the side, so that the pressure response generated by the pressure sensor will be lower than the actual pressure of the blood flow. Conversely, when the pressure sensor is configured to face the first chamber and second chamber, the blood flow will directly impact onto the sensing surface, so that the pressure sensor is able to more accurately reflect the actual pressure of the blood flow.

Furthermore, as shown in FIG. 2, when the pair of pressure sensors 120a, 120b are arranged centrally on the pad 111 located between the leaflets of the valve, they are located at the central position of the blood flow path, at this position they are able to more accurately reflect the overall pressure of the atrium or ventricle, and thereby more accurately reflecting whether there is stenosis or regurgitation at the valves.

On the other hand, as illustrated in FIG. 2, since the pair of pressure sensors 120a, 120b are attached to the top surface 111a and bottom surface 111b of the pad 111 respectively, while the leaflets of the valve are located on the sides of the pad 111, therefore, the top surface 111a and the bottom surface 111b of the pad will not contact with the leaflets of the valve when the valve closes. Consequently, the leaflets are prevented from interfering with the pressure sensors 120a, 120b, so that the accuracy of the pressure sensors 120a, 120b sensing the blood pressure is improved, and thus whether there is stenosis or regurgitation at the valve is more accurately and more timely reflected.

Consequently, by using the pair of pressure sensors 120a, 120b respectively attached to the top surface 111a and the bottom surface 111b of the pad 111, whether the valve has stenosis or regurgitation issues after the cardiac implant 100 is implanted is able to be reliably detected, so that the situation of the patient's postoperative recovery and development of the condition of the patient are able to be accurately monitored.

It should be noted that, although in the foregoing embodiments, the cardiac implant 100 includes the pair of sensors 120a, 120b, in other implementations, the cardiac implant 100 may include merely the sensor 120a attached to the top surface 111a, or may include merely the sensor 120b attached to the bottom surface 111b. Certainly, in other examples, the cardiac implant 100 may include more sensors.

It should also be noted that, although in the foregoing embodiment, the pair of sensors 120a, 120b are respectively attached to the top surface 111a and the bottom surface 111b of the pad 111, in other examples, the pair of sensors 120a, 120b may also be attached to other portions of the pad 111. For example, in some examples, one or more sensors may be attached to the sides of pad 111.

Continuing referring to FIG. 1 and FIG. 2, the pad 111 further includes a pair of coaptation surfaces 111c, 111d. The pair of coaptation surfaces 111c, 111d are configured to face the pair of leaflets FL, SL, respectively. As the cardiac cycle changes, each coaptation surface of the pair of coaptation surfaces 111c, 111d periodically coaptates and separates from the leaflet it faces, and thereby enabling the valve to be periodically opened and closed. Specifically, when one of the coaptation surfaces 111c,111d coaptates its corresponding leaflet, a coaptation length between them may range from 6 mm to 12 mm. In particular, the leaflet FL may be the anterior leaflet, and the leaflet SL may be the posterior leaflet, and the pad 111 is configured to be closer to the leaflet SL than to the leaflet FL. Specifically, the coaptation surfaces 111c, 111d may be smooth transition curved surfaces to reduce resistance to the blood flow.

When the second chamber is in the diastole, the pair of leaflets FL, SL move away from each other to form passages between the pair of leaflets FL, SL and the pair of coaptation surfaces 111c, 111d, respectively. At this time, the valve opens to allow the blood flow to pass through these passages from the first chamber into the second chamber. When the second chamber is in the systole, the pair of leaflets FL, SL move toward each other to make the pair of leaflets FL, SL coaptate the pair of coaptation surfaces 111c, 111d, respectively. At this time, the valve closes to prevent the blood flow flowing from the second chamber back to the first chamber. It should be noted that, in the present application, when a leaflet coaptates a coaptation surface, the leaflet and the coaptation surface may be in contact with each other to prevent the blood flow from flowing therebetween.

In this implementation, the pad 111 does not affect or minimally affects the movement of the pair of leaflets FL, SL, so that the pair of leaflets FL, SL are able to maintain their original physiological functions. That is, in this implementation, as the cardiac cycle changes, the pair of leaflets FL, SL are able to naturally and periodically move toward and away from each other. Therefore, by using the treatment device according to this implementation, there will be minimally or even no adverse effects on the structure and function of the pair of leaflets FL, SL. In other words, the cardiac implant 100 provided by the present application has no restriction or hindrance on the natural movement (closing, opening) of the valve, and it is not associated with the supporting structure of the valve, so that the corresponding adverse effect is avoided.

Continuing referring to FIG. 1 and FIG. 2, the treatment device 110 may also include a support member 112. After the cardiac implant 100 is implanted into a patient's heart, the support member 112 is located in the first chamber and is connected to the pad 111, and thereby positioning the pad 111 between the pair of leaflets SL, FL. By means of the support member 112 located in the first chamber, the pad 111 is able to be reliably positioned between the pair of leaflets FL, SL. In particular, the connection position between the support member 112 and the pad 111 is configured to be adjustable to allow the position of the pad between FL and SL to be adjusted.

Continuing referring to FIG. 1 and FIG. 2, the support member 112 may be annular in shape, and the pad 111 is centrally disposed on a side of the support member 112 along a first radial direction of the support member 112, such that the pad 111 is centrally positioned between the pair of leaflets FL, SL of the valve. The length direction of the pad 111 is consistent with the first radial direction, and the width direction and height direction of the pad 111 are perpendicular to the first radial direction. Along the length direction of the pad 211, the pad 211 has two side surfaces which are arc-shaped curved surfaces configured to fit with the leaflets. After the cardiac implant 100 is implanted into the patient's heart, the support member 112 may be placed at the valve annulus of the heart. In some embodiments, the support member 112 may have a mesh frame structure. During delivery, the support member 112 may firstly be folded to reduce its volume. After being delivered to a target position, it may be unfolded again.

It may be understood that, the implementation of the support member 112 is not limited to the above, as long as it is able to position the pad 111 between the pair of leaflets FL, SL of the valve. For example, in some embodiments, the support member 112 may also be semi-annular or partially annular in shape.

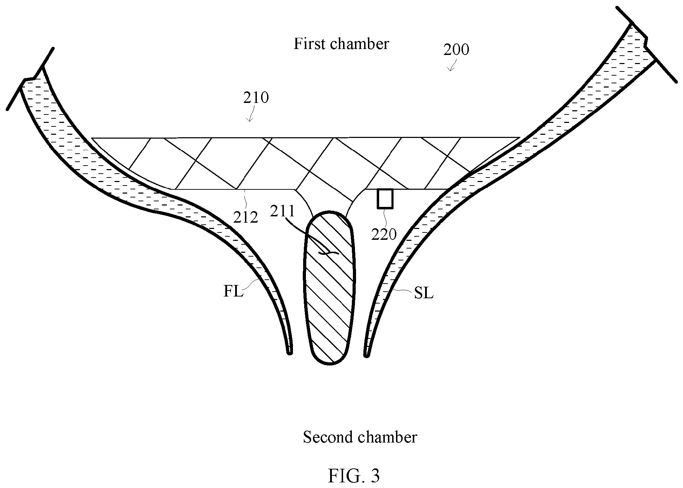

FIG. 3 is a schematic structural diagram illustrating a cardiac implant 200 according to another embodiment of the present application that is implanted in a patient's heart. The cardiac implant 200 is substantially the same as the cardiac implant 100. For the sake of brevity, the similarities will not be repeated herein.

Referring to FIG. 3, the cardiac implant 200 includes a treatment device 210. The treatment device 210 includes a pad 211 and a support member 212. The pad 211 is substantially the same as the pad 111 in the aforementioned embodiment, and the support member 212 is substantially the same as the support member 112 in the aforementioned embodiment. Relevant descriptions of the pad 211 and the support member 212 may refer to the descriptions of the pad 111 and the support member 112 in the aforementioned embodiments.

The cardiac implant 200 also includes a sensor 220. The sensor 220 is attached to the support member 212 and is configured to sense physiological information of the patient. In particular, the sensor 220 may be a pressure sensor. Specifically, the sensor 220 may be attached to a portion of the support member 212 closer to the body surface, such as closer to the anterior, left, or posterior chest wall. In particular, the sensor 220 may be attached to the support member 212, and their relative positions may be configured such that the support member 212 is not located between the sensor 220 (at least the communication portion of the sensor 220) and an external device in communication with the sensor 220. In this way, interference from the support member 212 (especially when it is made of metal) on wireless communication between the external device and the sensor is minimized.

As shown in FIG. 3, both the sensor 220 and the pad 211 are located at the side (that is the lower side in the figure) that closer to the second chamber in a direction from the first chamber to the second chamber, and the sensor 220 is offset from the pad 211 in the radial direction of the support member 212. It may be understood that, the support member 212 (or its main body portion) is generally annular (closed or unclosed) or has a circular surface facing the second chamber. Therefore, the orientation term "radial" in the present application may be relative to this annular shape or circle shape.

Specifically, referring to FIG. 3, the sensor 220 and the pad 211 are offset from each other along the direction from one leaflet SL to the other leaflet FL, such that when the support member 212 is implanted between the leaflets of the valve, the sensor 220 is located between the leaflet SL and the pad 211. The sensor 220 is a pressure sensor, and an end of the sensor 220 is fixed to a lower edge or a bottom surface of the support member 212 (that is, the edge or surface on the lower side of the support member 212), and the other end of the sensor 220 faces the second chamber, so that this facilitates sensing the pressure of blood flow that flows from the second chamber into the first chamber.

It may be understood that, the other end of the sensor 220 has a sensing surface. When the valve is closed, the sensing surface is located in a region formed by the lower side of the support member 212, the leaflet SL and the pad 211 and faces the second chamber. This ensures that if regurgitation that flows from the second chamber to the first chamber exists between the leaflet SL and the pad 211, the regurgitant will nearly directly impact on to the sensing surface of the sensor 220, so that the sensor 220 senses pressure information due to the blood flow. By arranging the sensor 220 on the support member 212, between the leaflet SL and the pad and the sensing surface facing toward the second chamber, the sensor is able to quickly and accurately sense the blood pressure caused by the regurgitation. If the sensor is set to be in other orientations (for example, facing the first chamber), it might not be able to, or would more difficult/slower to detect the regurgitation from the second chamber due to pressure and directional changes of blood flow. In the present embodiment, however, even there is minimal regurgitation between the first and second chamber, the sensor 220 may sensitively capture it.

It should be understood that, although merely one sensor 220 is shown in FIG. 3, the cardiac implant 200 may include a plurality of sensors. The plurality of sensors may be attached to different portions of the support member 212. For example, one sensor may be disposed at a lower edge of the support member 212 and between the leaflet FL and the pad 211, and another sensor may be disposed at the lower edge of the support member 212 and between the leaflet SL and the pad 211. In the case that the valve is a tricuspid valve, there may be one sensor disposed at the lower edge of the support member 212 and between the third leaflet and the pad 211. Alternatively, some of the plurality of sensors may be attached to the support member 212, and others may be attached to the pad 211.

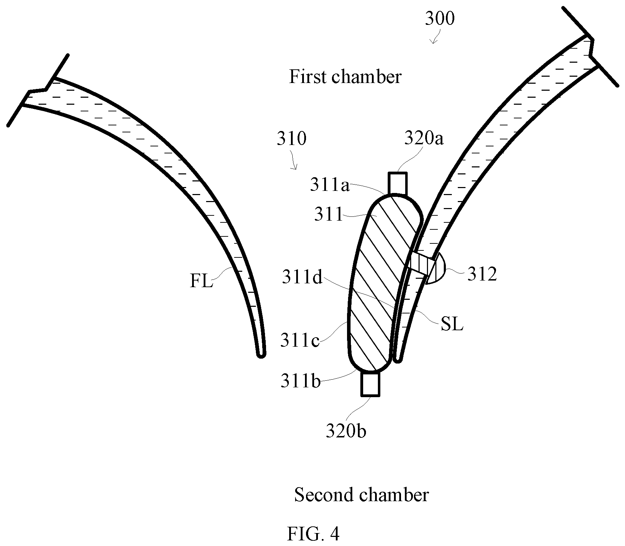

FIG. 4 is a schematic structural diagram illustrating a cardiac implant 300 according to another embodiment of the present application that is implanted in a patient's heart. The cardiac implant 300 is substantially the same as the cardiac implant 100. For the sake of brevity, the similarities will not be repeated.

Referring to FIG. 4, the cardiac implant 300 includes a treatment device 310 and a pair of sensors 320a, 320b. The treatment device 310 includes a pad 311 having a top surface 311a and a bottom surface 311b that face the first chamber and the second chamber, respectively. The pair of sensors 320a,320b may be a pair of pressure sensors 320a, 320b, and the pair of pressure sensors 320a, 320b may be attached to the top surface 311a and bottom surface 311b, respectively.

It should be noted that, in other examples, the cardiac implant 300 may include merely one of the pair of sensors 320a, 320b. It should also be noted that, in other examples, the sensors of cardiac implant 300 may be attached to positions on a place other than the top surface 311a and bottom surface 311b, such as be attached to the support frame. It should also be noted that, in other examples, the sensors of cardiac implant 300 may be sensors that are capable of sensing other physiological information.

The cooperation mode between the pad 311 and the pair of leaflets FL, SL differs from that between the pad 111 and the pair of leaflets FL, SL in the aforementioned embodiments. Specifically, the pad 311 includes a pair of coaptation surfaces 311c, 311d. After the cardiac implant 300 is implanted into the patient's heart, the pad 311 is configured to be located between the pair of leaflets FL, SL and follow the movement of leaflet SL, and the pair of coaptation surfaces 311c, 311d are configured to respectively face the pair of leaflets FL, SL. As the pad 311 follows the movement of leaflet SL, the coaptation surface 311d is configured to maintain coaptating the leaflet SL (for example, a coaptation length between them may range from 6 mm to 12 mm), and the coaptation surface 311c is configured to periodically coaptate and separate from leaflet FL. In other words, with changes in the cardiac cycle, the coaptation surface 311d remains in constant coaptate the leaflet SL, while the coaptation surface 311c periodically coaptates and separates from the leaflet FL.

In this implementation, the pad 311 does not affect or minimally affects the movement of the pair of leaflets FL, SL, so that the pair of leaflets FL, SL, particularly the leaflet FL, are able to maintain their original physiological functions. That is, in this implementation, as the cardiac cycle changes, the pair of leaflets FL, SL are able to naturally and periodically move toward and away from each other. Consequently, it may be seen that, this implementation has minimal adverse effects on the structure and function of the leaflets FL, SL.

There are many ways to position the pad 311 between the pair of leaflets FL, SL and to make the pad 311 move with the leaflet SL, which is not specifically limited in the present application. As an example, continuing referring to FIG. 4, the treatment device 310 also includes a pin 312 or other fixing member, this kind of fixing member is connected to the pad 311 and used for connecting the pad 311 to the leaflet. Specifically, the pin 312 may penetrate through the leaflet SL to attach the pad 311 to leaflet SL, and thereby locating the pad 311 between the pair of leaflets FL, SL and enabling it to follow the movement of leaflet SL. In this way, as the cardiac cycle changes, the coaptation surface 311d facing leaflet SL is able to maintain coaptating the leaflet SL, and the coaptation surface 311c facing leaflet FL is able to periodically coaptate and separate from the leaflet FL with the pad 311 following the movement of leaflet SL.

It should be noted that, the treatment device 310 may include merely one pin 312, or it may include a plurality of pins 312.The present disclosure does not specifically limit this. The pin 312 may be integrally formed with the pad 311, and the two may also be two independent members, which are not specifically limited in the embodiments of the present application.

Although the pad 311 in FIG. 4 is disposed on the leaflet on a side, the top surface 311a and bottom surface 311d of the pad 311 still face the first chamber and the second chamber, respectively. Consequently, the pressure sensors 320a, 320b on the top surface 311a and bottom surface 311d are also disposed to face the first chamber and the second chamber, respectively. Therefore, the blood flow will directly impact onto the pressure sensors 320a, 320b, so that the pressure sensors 320a, 320b are able to accurately reflect the pressure of the blood flow.

As shown in FIG. 4, since the sensors 320a, 320b are attached to the top surface 311a and bottom surface 311b of the pad 311, respectively, the sensor 320a is away from the leaflet SL in a manner facing the first chamber, and the sensing surface of sensor 320b is away from a distal end of the leaflet SL in a manner facing the second chamber, so that the pressure sensors 320a, 320b will not contact with the leaflets of the valve when the valve closes. This prevents the valve from interfering with the pressure sensors 320a, 320b, and thereby the accuracy of the blood pressure sensed by the pressure sensors 320a, 320b is further improved.

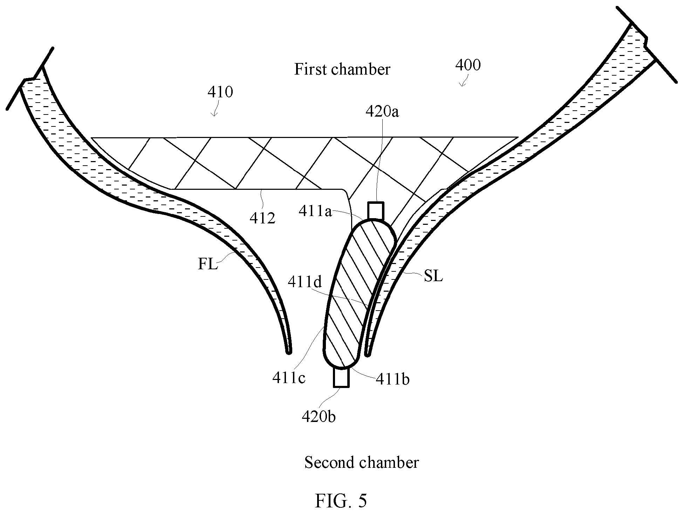

FIG. 5 is a schematic structural diagram illustrating a cardiac implant 400 according to another embodiment of the present application that is implanted in a patient's heart. The cardiac implant 400 is substantially the same as the cardiac implant 100. For the sake of brevity, the similarities will not be repeated.

Referring to FIG. 5, the cardiac implant 400 includes a treatment device 410 and a pair of sensors 420a, 420b. The treatment device 410 includes a pad 411 and a support member 412 for positioning the pad 411 between the pair of leaflets FL, SL. The support member 412 is substantially the same as the support member 112 in the aforementioned embodiments. The pad 411 has a top surface 411a facing the first chamber and a bottom surface 411b facing the second chamber. The pair of sensors 420a, 420b may be a pair of pressure sensors 420a, 420b, which may be respectively attached to the top surface 411a and bottom surface 411b. The difference lies in that the pad 411 is offset to a side of the support member 412, and in addition, the connection between the support member 412 and the pad 411 is configured to be movable to allow the pad 411 to move with leaflet SL during the cardiac cycle.

It should be noted that, in other examples, the cardiac implant 400 may include merely one of the pair of sensors 420a, 420b. It should also be noted that, in other examples, the sensors of the cardiac implant 400 may be attached to positions on pad 411 other than the top surface 411a and bottom surface 411b. For instance, in some examples, the cardiac implant 400 may also include at least one sensor attached to the support member 412, and the at least one sensor is disposed at a lower edge of the support member 412 and faces the second chamber, and is disposed between the pad 411 and the leaflet FL. It should be noted that, in other examples, the sensors of cardiac implant 400 may also be sensors that are capable of sensing other physiological information.

The pad 411 also includes a pair of coaptation surfaces 411c, 411d. The pair of coaptation surfaces 411c, 411d are configured to respectively face the pair of leaflets FL, SL. After the cardiac implant 400 is implanted into the patient's heart, the pad 411 is rigidly connected to the support member 412 (that is, connected in a manner that prevents relative movement), and thereby maintaining the pad 411 at a preset position between the pair of leaflets FL, SL while keeping the coaptation surface 411d coaptating the leaflet SL. This preset position is configured such that the coaptation surface 411c is able to periodically coaptate and separate from the leaflet FL with the movement of the leaflet FL, and the leaflet FL coaptates the coaptation surface 411c during the systole and achieves a certain coaptation length (for example, 6 mm~12 mm), so that effective closure of leaflets is increased, and thus the blood flow that flows from the second chamber to the first chamber is prevented. During diastole, the leaflet FL opens to allow the blood to flow from the first chamber through a passage between leaflet FL and the coaptation surface 411c into the second chamber. In particular, the pad 411 is configured to avoid causing stenosis during diastole due to an insufficient space between the leaflet FL and the coaptation surface 411c, such as by adjusting the thickness of pad 411 and/or its position relative to the leaflet FL.

In this implementation, since the pad 411 is held at a predetermined position between the pair of leaflets FL,SL and the coaptation surface 411d of the pad 411 remains coaptating the leaflet SL, the pad 411 will affect the movement of the leaflet SL, and thereby causing the leaflet SL to reduce or lose its original physiological function. Compared with the aforementioned implementation, if a treatment device according to this implementation is used, it may have certain influence on the structure and function of the leaflet SL after a longer period of use. Without doubt, in terms of the overall function of the valve, the leaflet FL plays a greater role relative to the leaflet SL.

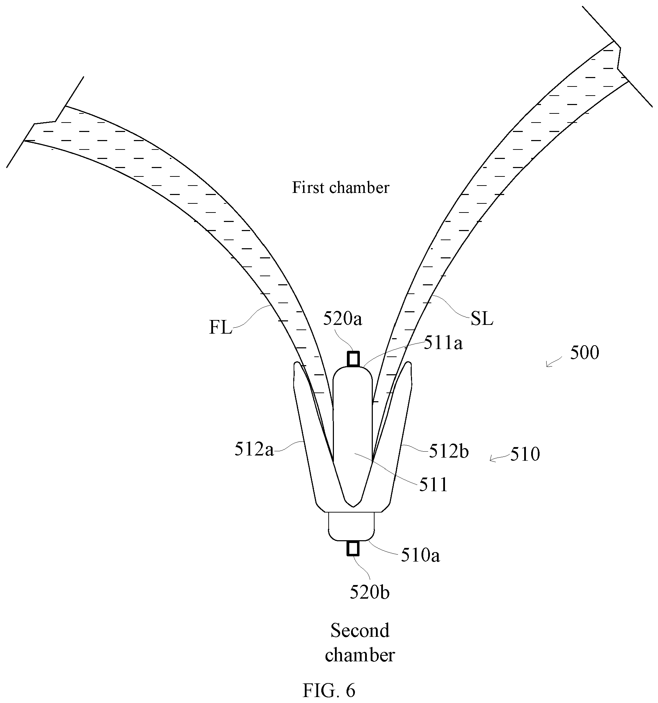

FIG. 6 is a schematic structural diagram illustrating a cardiac implant according to further another embodiment of the present application that is located in a patient's heart.

Referring to FIG. 6, the cardiac implant 500 includes a treatment device 510 and a pair of sensors 520a,520b. The treatment device 510 includes a coaptation member 511 and a pair of blades 512a,512b which are located on two opposite sides of the coaptation member 511. In other embodiments, the treatment device may also include three blades for holding three leaflets of the valve, respectively.

After implanting the cardiac implant 500 in a patient's heart, the pair of blades 512a,512b is configured to hold the coaptation member 511 between the pair of leaflets FL,SL. In one example, each blade may have a clip-like structure to clip the corresponding leaflet, and thereby positioning the coaptation member 511 between the pair of leaflets FL,SL. In another example, each blade may cooperate with the coaptation member 511 to clip the corresponding leaflet between them, and thereby positioning the coaptation member 511 between the pair of leaflets FL,SL. It should be understood that, the specific implementation of the pair of blades 512a,512b may refer to the related technology. For the sake of brevity, it will not be repeated herein.

When the second chamber is in diastole, the pair of leaflets FL,SL form a double orifice on two side of the coaptation member 511; and at this time, the valve is open, so that allowing the blood to flow from the first chamber into the second chamber. When the second chamber is in systole, the pair of leaflets FL,SL coaptate appropriately on the two side of the coaptation member 511; and at this time, the valve closes properly, and thereby regurgitation of the blood is avoided.

The coaptation member 511 has a top surface 511a facing the first chamber, and the treatment device 510 has a bottom surface 510a facing the second chamber. The pair of sensors 520a,520b may be a pair of pressure sensors. The pair of pressure sensors 520a,520b may be respectively attached to the top surface 511a of the coaptation member and the bottom surface 510a of the treatment device 510 to sense the blood pressure in the first chamber and second chamber, respectively.

The pressure sensors 520a,520b are capable of sensing the blood pressure throughout the entire cardiac cycle in the first chamber and second chamber, respectively, so that hemodynamic information having diagnostic value is provided to the physician. This information plays a crucial role in assessing the overall function of the heart, as well as the working conditions of the valve and the implant. Meanwhile, the pressure information is also able to assist the physician in evaluating a placement position of the implant during the implantation process, and in making real-time assessments of whether there is any obstruction to blood flow from the first chamber into the second chamber during diastole. In this way, the success of the implantation operation is better ensured, and thus better clinical effect is achieved.

When the valve is open, the blood flow from the first chamber into the second chamber impacts the pressure sensor 520a attached to the top surface 511a (at a diaphragm of the pressure sensor 520a) nearly perpendicularly. This enables the pressure sensor 520a to sense physiological information (that is, pressure information) that is able to relatively accurately reflect whether the blood flow from the first chamber to the second chamber is normal, and thereby being able to relatively accurately indicate whether there is any stenosis of the valve after implanting the cardiac implant 500.

When the valve closes, if there is still regurgitation from the second chamber to the first chamber, the regurgitation will impact the pressure sensor 520b attached to the bottom surface 510a (at a diaphragm of the pressure sensor 520b) nearly perpendicularly, and thus the physiological information and changes thereof due to blood flow are sensed by the sensor 520b (that is, pressure information), including whether there is any regurgitation from the second chamber to the first chamber.

Furthermore, as shown in FIG. 6, when the pressure sensors 520a,520b are set to be centered on the coaptation member 511 between the leaflets of the valve, the pressure sensors 520a,520b are located on a center position of a path of the blood flow. At this position, the true pressure of the atrium or ventricle is able to be most accurately reflected.

On the other hand, as shown in FIG. 6,since the pressure sensors 520a,520b are attached to the top surface 511a and bottom surface 511b of the pad 511 respectively, when the valve closes, the pad 511 will not contact with the leaflets of the valve, thereby avoiding interference from the valve on the pressure sensors 520a,520b, and thus the accuracy of the pressure sensors 520a,520b in sensing blood pressure is further improved.

It can be seen that, by the pair of pressure sensors 520a,520b attached to the top surface 511a and bottom surface 510a of the coaptation member 511 respectively, it is possible to relatively accurately detect whether there is any stenosis or regurgitation at the valve after implanting the cardiac implant 500, and thereby the patient's postoperative recovery and disease progression is more accurately monitored.

It should be noted that, in other examples, the cardiac implant 500 may include merely the sensor 520a attached to the top surface 511a, or it may include merely the sensor 520b attached to the bottom surface 510a. Certainly, in other examples, the cardiac implant 500 may also include more sensors.

It should also be noted that, in other examples, one or more sensors may be attached to other portions of the treatment device 500. For example, in some examples, one or more sensors may be attached to a side surface of the coaptation member 511.

It should also be noted that, in other examples, the sensors of the cardiac implant 500 may be sensors capable of sensing other physiological information.

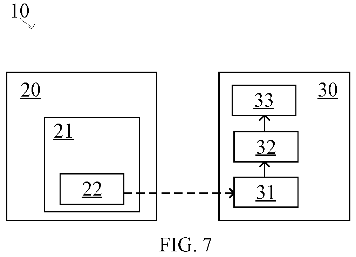

In another embodiment of the present invention, a cardiac implant system 10 is also provided. Referring to FIG. 7, the implant system 10 includes a cardiac implant 20 and an external device 30. The cardiac implant 20 may be any one of the cardiac implants 100, 200, 300,400 or 500 described in the foregoing embodiments. The sensor 21 of the cardiac implant 20 has a wireless transmission unit 22. The external device 30 includes a wireless reception unit 31, an analysis unit 32 and an alarm unit 33 that are in communication connection with each other. The wireless reception unit 31 receives pressure value information from the wireless transmission unit 22 of the sensor 21. The analysis unit 32 determines whether to send an alarm signal to the alarm unit 33 based on the pressure value information. Upon receiving the alarm signal, the alarm unit 33 issues an alarm message through any one or a combination of a pattern, text or sound to alert the doctor with the abnormal opening/closing conditions of the valve. Consequently, the doctor is able to track and determine the post-implantation conditions of the implant 20 based on the sensed pressure value information and promptly carry out the subsequent treatment.

Specifically, for the cardiac implants 100, 300, 400 or 500, it may be configured such that when a pressure difference between the two sensors 120a, 120b (or between two sensors 320a, 320b, or between two sensors 420a, 420b, or between two sensors 520a, 520b) exceeds a preset threshold (for example, 5 mmHg) within a specified time period (indicating possible valve stenosis), the analysis unit 32 send an alarm signal to the alarm unit 33. For the cardiac implant 200, it may be configured such that when the change in the pressure value is greater than a preset threshold within a specified time period (indicating possible valve regurgitation), the analysis unit 32 send an alarm signal to the alarm unit 33. This predetermined threshold may be determined based on pressure values obtained under normal valve closure conditions.

It should be understood that, the term "include" and its variations used in the present application are open-ended inclusions, that is, it means "including but not limited to". The term "one embodiment" means "at least one embodiment", and the term "another embodiment" means "at least one another embodiment."

It should be understood that, although terms such as "first" or "second" may be used in the present application for describing various elements (for example, the first chamber and the second chamber), but these elements are not limited by these terms, and these terms are merely used for distinguishing one element from another.

It should be noted that, the various specific technical features (elements) described in the above specific embodiments may be combined in any suitable manner without contradiction. To avoid unnecessary repetition, the present application will not separately describe all possible combinations.

It should be understood that, multiple members and/or portions may be provided by a single integrated member or portion. Alternatively, a single integrated member or portion may be divided into separate multiple members and/or portions. The use of "a" or "an" to describe a member or portion is not intended to exclude other members or portions.

The foregoing descriptions are merely specific implementations of the present application, but the protection scope of the present application is not limited thereto. Any person skilled in the art may conceive of modifications or substitutions within the technical scope disclosed in the present application, and such modifications or substitutions should be covered by the protection scope of the present application. Therefore, the protection scope of the present application shall be subject to the scope defined by the claims.

## Claims

1. A cardiac implant, adapted to be implanted into a heart, the heart comprising a first chamber, a second chamber and a valve, the valve being configured to periodically allow blood to flow from the first chamber to the second chamber, wherein the cardiac implant comprises a treatment device and at least one sensor, the treatment device is configured to repair a function of the valve to prevent the blood from flowing back from the second chamber into the first chamber, and the at least one sensor is attached to the treatment device and is configured to sense a physiological information of the patient.

2. The cardiac implant according to claim 1, wherein the valve comprises at least two leaflets, the treatment device comprises a pad, the pad is configured to be located between the at least two leaflets and cooperate with the at least two leaflets to periodically open and close the valve, and the at least one sensor is attached to the pad.

3. The cardiac implant according to claim 2, wherein the pad has a top surface configured to face the first chamber, the at least one sensor comprises a pressure sensor, and the pressure sensor is attached to the top surface of the pad to sense a blood pressure in the first chamber; and/or
the pad has a bottom surface configured to face the second chamber, the at least one sensor comprises a pressure sensor, and the pressure sensor is attached to the bottom surface of the pad to sense a blood pressure in the second chamber.

4. The cardiac implant according to claim 2, wherein the treatment device further comprises a support member or a fixing member connected to the pad, the fixation member is configured to attach the pad to one of the at least two leaflets, and the support member is configured to position the pad between the at least two leaflets.

5. The cardiac implant according to claim 1, wherein the valve comprises at least two leaflets, the treatment device comprises a support member and a pad, the support member is configured to be located in the first chamber and be connected to the pad to position the pad between the at least two leaflets, the at least one sensor comprises a pressure sensor, and the pressure sensor is attached to the support member.

6. The cardiac implant according to claim 5, wherein the pad is movably connected to the support member; or, the pad is biased at a side of the support member to maintain fitting with one of the at least two leaflets of the valve; or, the pad is centrally positioned at a side of the support member to be centrally located between the at least two leaflets.

7. The cardiac implant according to claim 5, wherein after the cardiac implant is implanted in the heart, the pad and the pressure sensor are both located on a side of the support member closer to the second chamber in a direction from the first chamber to the second chamber, and the pressure sensor is provided on the support member and is offset from the pad in a radial direction of the support member.

8. The cardiac implant according to claim 7, wherein an end of the pressure sensor is fixed to the support member, and the other end of the pressure sensor has a sensing surface, the sensing surface is configured to face the second chamber to sense a pressure of the blood flowing from the second chamber into the first chamber.

9. The cardiac implant according to claim 1, wherein the cardiac valve comprises at least two leaflets, the treatment device comprises a coaptation member and at least two blades located on two sides of the coaptation member respectively, and the at least two blades are configured to position the coaptation member between the at least two leaflets.

10. The cardiac implant according to claim 9, wherein the coaptation member has a top surface configured to face the first chamber, the at least one sensor includes a pressure sensor, the pressure sensor is attached to the top surface of the coaptation member to sense a blood pressure in the first chamber; and/or
the treatment device has a bottom surface configured to face the second chamber, the at least one sensor includes a pressure sensor, and the pressure sensor is attached to the bottom surface of the treatment device to sense a blood pressure in the second chamber.

11. The cardiac implant according to claim 1, wherein the at least one sensor includes a pressure sensor, and a sensing surface of the pressure sensor is configured to face the first chamber or the second chamber.

12. An implant system, comprising the cardiac implant according to any one of claims 1 to 15 and an external device, the at least one sensor comprises a wireless transmission unit, the external device comprises a wireless reception unit, and the wireless reception unit receive pressure value information from the wireless transmission unit of the at least one sensor.

13. The implant system according to claim 12, wherein the external device further comprises an analysis unit and an alert unit; the analysis unit receives the pressure value information from the wireless reception unit and determines whether to send an alert signal to the alert unit based on the pressure value information; and after the alert unit receives the alert signal, the alert unit sends an alert message.
